(19) Europäisches Patentamt
European Patent Office
Office européen des brevets

(11) Numéro de publication: **0 147 348**
**B1**

# (12) FASCICULE DE BREVET EUROPÉEN

(45) Date de publication du fascicule du brevet: **05.11.86**

(51) Int. Cl.⁴: **C 07 C 91/12,** A 61 K 31/13 // C07D303/36

(21) Numéro de dépôt: **84450026.4**

(22) Date de dépôt: **13.11.84**

(54) **Nouveaux cycloalkylamino-1 tertiobutylamino-3 propanols-2, leur méthode de préparation et leur application en thérapeutique.**

(30) Priorité: **22.12.83 FR 8320748**

(43) Date de publication de la demande: **03.07.85 Bulletin 85/27**

(45) Mention de la délivrance du brevet: **05.11.86 Bulletin 86/45**

(84) Etats contractants désignés: **BE CH DE GB IT LI LU NL**

(56) Documents cité:
**CHEMICAL ABSTRACTS, vol. 69, no. 19, 4 novembre 1968, page 7170, abrégé 76809r, Columbus, Ohio, US; G. FERRARI et al.: "Beta-Adrenergic blocking drugs. III. 1-Aryloxy- and 1-arylamino-3-amino-2-propanols"**
**JOURNAL OF HETEROCYCLIC CHEMISTRY, vol. 6, no. 3, juin 1969, pages 273-277; V.R. GAERTNER: "Ring-opening nucleophilic alkylations by tertiary azetidines"**

(73) Titulaire: **SOCIETE CORTIAL S.A., 7 rue de l'Armorique, F-75015 Paris (FR)**

(72) Inventeur: **Boyer, Chantal, 18 route de Soulac Beaulieu, F-33290 Le Pian Médoc (FR)**
Inventeur: **Colleter, Jean- Claude, Rue du Général de Gaulle Ludon Médoc, F-33290 Blanquefort (FR)**
Inventeur: **Creuzet, Marie- Hélène, Résidence Jardin de Gambetta T3, F-33000 Bordeaux (FR)**
Inventeur: **Feniou, Claude, 5 rue du Général Guillomat, F-33600 Pessac (FR)**
Inventeur: **Laguerre, Michel, Résidence la Fleurière Bât A Appt 15, F-33170 Gradignan (FR)**
Inventeur: **Pontagnier, Henri, 21 rue Edouard Vaillant, F-33600 Pessac (FR)**
Inventeur: **Prat, Gisèle, Hameau de Noailles Villa 18, F-33400 Talence (FR)**

(74) Mandataire: **Tajan, Marie- Thérèse, LABORATOIRES SARGET Avenue du Président JF Kennedy, F-33701 Mérignac (FR)**

**EP 0 147 348 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

La présente invention concerne de nouveaux cycloalkylamino-1 tertiobutylamino-3 propanols-2, leur méthode de préparation et leur application en thérapeutique notamment en tant qu'agents antimicrobiens.

Les produits selon la présente invention ont pour formule générale

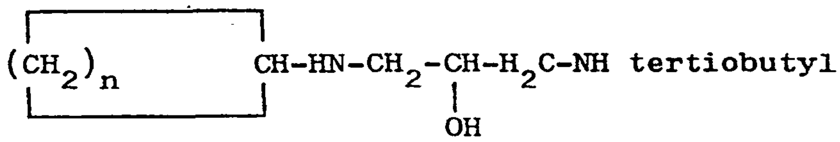

(I)

dans laquelle n représente un entier pouvant prendre l'une quelconque des valeurs comprises entre 6 et 11 et peuvent être sous forme de base libre ou de sels pharmaceutiquement acceptables.

Les produits de formule générale (I) sont nouveaux. Seul leur homologue cyclohexylique (produit de formule (I) avec n = 5) a déjà été synthétisé (GAERTNER V.R., J. Heterocycl. Chem., 1969, 6, 3, 273-7) au cours d'un travail visant à étudier l'alkylation nucléophile décyclisante par des azétidines tertiaires. Aucune application n'est connue pour ce produit.

Nous avons maintenant découvert que les produits selon la formule (I) présentent des propriétés pharmacologiques notamment antiagrégantes plaquettaires et antimicrobiennes permettant leur utilisation en thérapeutique.

Les produits selon la formule (I) sont préparés de façon générale en deux étapes: on fait réagir l'amine

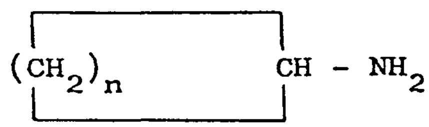

(où n représente un entier pouvant prendre l'une quelconque des valeurs comprises entre 6 et 11) et l'épichlorhydrine

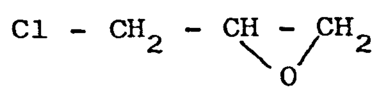

dans un solvant tel que l'éthanol à la température d'ébullition du solvant; le produit obtenu

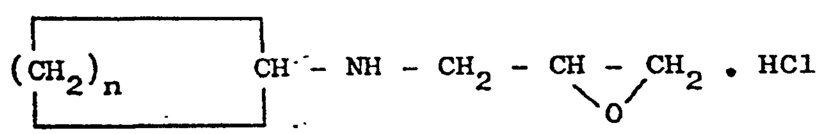

donne, par réaction avec la tertiobutylamine, dans un solvant tel que l'éthanol, à la température d'ébullition du solvant, le dérivé de formule (I) attendu.

La présente invention va être décrite plus précisément dans les exemples suivants sans que ceux-ci ne puissent toutefois en limiter la portée.

### EXEMPLE 1

Dichlorhydrate du cyclooctylamino-1 tertiobutylamino-3 propanol-2; nom de code COR37 07C; dichlorhydrate du produit de formule (I) avec n = 7.

On porte au reflux dans de l'éthanol absolu pendant 24 heures 1 mole de cyclooctylamine et 1,1 mole d'épichlorhydrine. On obtient ainsi l'époxyde

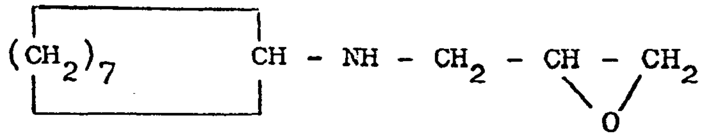

sous forme de chlorhydrate. Celui-ci est mis en solution dans l'éthanol absolu et porté au reflux pendant 24 heures en présence d'un grand excès de tertiobutylamine. Après évaporation des solvants, le produit attendu est purifié sur colonne et recristallisé. La base est obtenue avec un rendement de 60 % puis transformée en dichlorhydrate ou COR37 07C.

Point de fusion du chlorhydrate environ 125° C avec décomposition. Spectre de RMN de la base: 1 singulet, 9 protons à 1,05 ppm, tBu 1 multiplet, 14 protons à 1,50 ppm $(CH_2)_7$; 1 multiplet, 4 protons à 2,5 ppm, 2 $CH_2N$; 1 multiplet, 2 protons à 3,6 ppm, CHO + CHN.

**EXEMPLE 2**

Dichlorhydrate du cyclododécylamino-1 tertiobutylamino-3 propanol-2; nom de code COR37 52C; dichlorhydrate du produit de formule (I) avec n = 11.

Ce dérivé est préparé selon l'exemple 1 à partir de dodécylamine. Rendement 38 %.

Spectre de RMN de la base libre: 1 singulet, 9 protons, à 1,05 ppm, tertiobutyl; 1 multiplet, 22 protons, à 1,3 ppm, $(CH_2)_{11}$ 1 multiplet, 5 protons, à 2,5 ppm, 5CH-N; 1 multiplet, 1 proton, à 3,6 ppm, CHO.

Les propriétés toxicopharmacologiques des produits faisant l'objet de la présente invention sont exposées ci-après.

La toxicité à été déterminée chez la souris. Le pourcentage de mortalité enregistré pour une administration de CO37 07C ou de CO37 52C est de 0 % pour 300 mg/kg administrés par voie orale, 0 % pour 100 mg/kg administrés par voie intrapéritonéale et 100 % pour 200 mg/kg administrés par voie intrapéritonéale.

L'activité antimicrobienne à été déterminée in vitro par la technique de dilution en tubes. Les concentrations inhibitrices minimales sont de 5 microg/ml pour les COR 37 07C et COR37 52C vis-à-vis de Staphylococcus aureus de 5 microg/ml pour le COR37 07C et de 20 microg/ml pour le COR 37 52C vis-à-vis d'Escherichia coli; de 20 microg/ml pour le COR37 07C pour Mycobacterium ranae; de 20 microg/ml pour le COR37 07C vis-à-vis de Pseudomonas aeruginosa; de 20 microg/ml pour les COR37 07C et COR37 52C vis-à-vis de Tichophyton mentagrophytes; de 5 microg/ml pour le COR 37 07C et de 20 microg/ml pour le COR37 52C vis-à-vis de Proteus vulgaris; de 5 microg/ml pour le COR37 52 C vis-à-vis de Klebsiella pneumonia.

Lors d'un autre essai effectué in vitro selon la norme NF T 72.151 les résultats ont été pour le COR37 07C les suivants:

Escherichia coli ATCC 10536 CMI = CMB = 4 microg/ml
Staphylococcus aureus ATCC 9144 - CMI = CMB = 4 microg/ml
Pseudomonas aeruginosa CNCM A 22 CMI = 128 microg/ml
CMB = 256 microg/ml
Streptococcus faecalis ATCC 10541 CMI = CMB = 4 microg/ml
Mycobacterium smegmatis CNCM 7326 CMI = CMB = 1 microg/ml

L'activité anticalcique a été appréciée in vitro; à la concentration de 10 microg/ml le COR37 07C diminue de plus de 40 % la force contractile de l'oreillette de cobaye stimulée électriquement; cet effet est inhibé par l'addition de calcium.

L'activité antiagrégante plaquettaire a été déterminée in vitro. Le COR37 07C entraine à 25 microg/ml 60 % d'inhibition de l'agrégation plaquettaire induite par $10^{-6}$M d'ADP dans du plasma de lapin riche en plaquettes; l'adénosine dans les mêmes conditions, entraine 64 % d'activité à 100 microg/ml.

Le COR37 07C à 5 microg/ml et le COR37 52C à 10 microg/ml entrainent 100 % d'inhibition de l'agrégation induite par 0,05 ml d'une préparation standard de collagène bovin dans du plasma de lapin riche en plaquettes. L'aspirine dans les mêmes conditions entraine 100 % d'inhibition à 10 microg/ml.

Compte tenu de leurs activités pharmacologiques jointes à une faible toxicité les produits faisant l'objet de la présente invention pourront être employés en thérapeutique humaine et vétérinaire. Compte tenu de leur activité antimicrobienne ils pourront être employés seuls ou en association par exemple en tant qu'agents antiseptiques ou désinfectants pour l'antisepsie des mains, l'antisepsie de la peau saine ou lésée et l'antisepsie des muqueuses et séreuses, ainsi que pour la désinfection des matériels et surfaces. Ils pourront être utilisés selon l'indication en association avec les excipients habituels par exemple sous forme de solutions aqueuses ou alcooliques, solutions moussantes, savons, pommades, comprimés gynécologiques, ovules, pansements imprégnés; les solutions pourront contenir à titre d'exemple de 0,1 à 10 % de principe actif et seront utilisées selon l'indication pures ou diluées; les formes gynécologiques (comprimés, ovules par

exemple) pourront contenir par exemple de 0,050 à 0,250 g de principe actif par formule unitaire. Ils pourront également être utilisés dans le traitement des infections générales. Compte tenu de leurs propriétés anticalciques les produits faisant l'objet de la présente invention seront utiles dans le traitement de l'insuffisance coronarienne; compte tenu de leurs propriétés antiagrégantes plaquettaires ils seront utiles dans le traitement des états d'hyperagrégabilité plaquettaire. Associés aux excipients habituels ils seront administrés par exemple par voie orale sous forme de dragées, comprimés, sirops, ampoules, par voie rectale sous forme de suppositoire, par voie intramusculaire ou intraveineuse. Les doses administrées varieront selon l'indication et le sujet de 1 à 100 mg/j en une à six prises pour la voie orale, de 1 à 100 mg/j en une ou deux prises pour la voie rectale, de 0,5 à 50 mg/j par injection pour les voies parentérales.

**Revendications**

1 - Nouveaux produits de formule générale

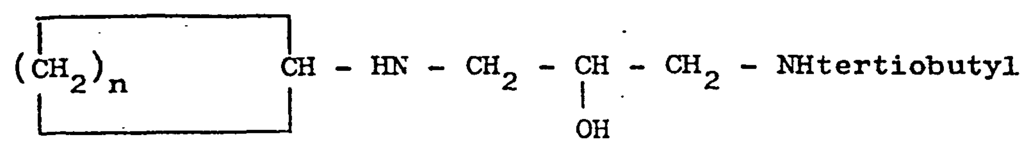

formule dans laquelle n représente un entier pouvant prendre l'une quelconque des valeurs comprises entre 6 et 11, produits pouvant être sous forme de base libre ou de sels pharmaceutiquement acceptables tels que les chlorhydrates.

2 - Nouveaux produits selon la revendication 1 caractérisés en ce que n = 7.

3 - Nouveaux produits selon la revendication 1 caractérisés en ce que n = 11.

4 - Méthode de préparation des produits selon les revendications 1 à 3 caractérisée en ce que l'on fait réagir l'amine

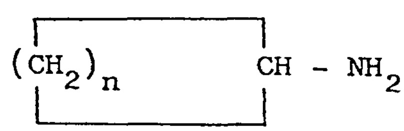

avec l'épichlorhydrine

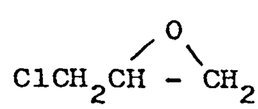

en présence d'un solvant tel que l'éthanol à la température d'ébullition du solvant et en ce que l'on fait réagir le dérivé ainsi obtenu avec la tertiobutylamine en présence d'un solvant tel que l'éthanol à la température d'ébulliton du solvant.

5 - Nouveau médicament caractérisé en ce que le principe actif est constitué par au moins un produit selon les revendications 1 à 3.

6 - Nouveau médicament selon la revendication 5 utile pour l'antisepsie de la peau saine et lésée, des muqueuses et des séreuses et pour l'antisepsie générale.

7 - Nouveau médicament selon la revendication 5 utile pour le traitement de l'insuffisance coronarienne.

8 - Nouveau médicament selon la revendication 5 utile pour le traitement des états d'hyperagrégabilité plaquettaire.

9 - Nouvelle composition caractérisée en ce qu'elle contient au moins un produit selon les revendications 1 à 3 utile pour la désinfection des matières inertes.

**Patentansprüche**

1. Neue Verbindungen der allgemeinen Formel

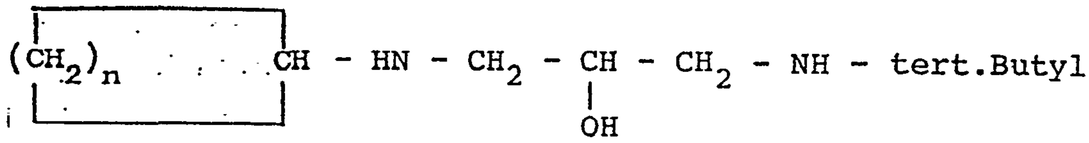

in der n eine ganze Zahl von 6 bis 11 bedeutet, in Form ihrer freien Basen oder pharmazeutisch verträglichen Salzen wie Chlorhydrate.

2. Verbindung nach Anspruch 1, dadurch gekennzeichnet, daß n = 7 ist.

3. Verbindung nach Anspruch 1, dadurch gekennzeichnet, daß n = 11 ist.

4. Verfahren zur Herstellung der Verbindungen nach einem der Ansprüche 1 bis 3, dadurch gekennzeichnet, daß man ein Amin der Formel

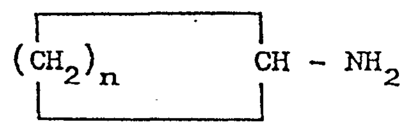

mit Epichlorhydrin

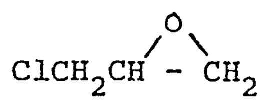

in Gegenwart eines Lösemittels wie Ethanol bei der Siedetemperatur des Lösemittels umsetzt und das so erhaltene Derivat mit tert.-Butylamin in Gegenwart eines Lösemittels wie Ethanol bei der Siedetemperatur des Lösemittels umsetzt.

5. Arzneimittel, dadurch gekennzeichnet, daß der Wirkstoff aus mindestens einer Verbindung nach einem der Ansprüche 1 bis 3 besteht.

6. Arzneimittel nach Anspruch 5 zur Verwendung für die Antisepsis der gesunden oder verletzten Haut, der Schleimhäute und der serösen Häute sowie für die allgemeine Antisepsis.

7. Arzneimittel nach Anspruch 5 zur Verwendung bei der Behandlung der Koronarinsuffizienz.

8. Arzneimittel nach Anspruch 5 zur Verwendung bei der Behandlung von Hyperaggregationszuständen der Blutplättchen.

9. Pharmazeutische Zusammensetzung, dadurch gekennzeichnet, daß sie mindestens eine Verbindung nach einem der Ansprüche 1 bis 3 enthält und zur Verwendung bei der Desinfektion von inerten Materialien bestimmt ist.

**Claims**

1. New products with the general formula

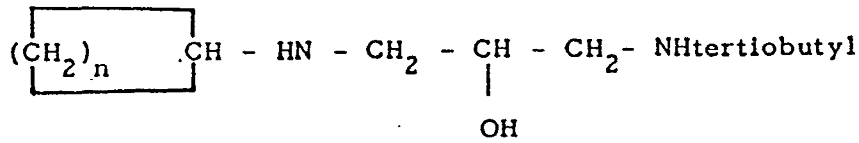

wherein n is an integral number which may take any value lying between 6 and 11, which products may lie in the form of free base or pharmaceutically-suitable salts, such as hydrochlorates.

2. New products as defined in claim 1, in which n = 7.

3. New products as defined in claim 1, in which n = 11.

4. Method for preparing products as defined in claims 1 to 3, which comprises reacting the amine

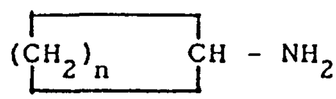

with the epichlorohydrine

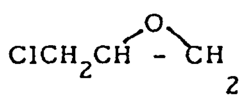

in the presence of a solvent such as ethanol at the solvent boiling temperature, and reacting the resulting derivative with tertiobutylamine in the presence of a solvent such as ethanol at the solvent boiling temperature.

5. New medicament, in which the active constituent is comprised of at least one product as defined in claims 1 to 3.

6. New medicament as defined in claim 5, useful for antisepticizing healthy and injured skin, mucous membranes and serous membranes, and for general antisepsis.

7. New medicament as defined in claim 5, useful for treating coronary deficiency.

8. New medicament as defined in claim 5, useful for treating plaquette hyperaggregativeness conditions.

9. New compound, which contains at least one product as defined in claims 1 to 3, useful for disinfecting inert materials.